# EUROPEAN PATENT APPLICATION

(11) **EP 4 699 540 A1**
(43) Date of publication of application: **25.02.2026**
(21) Application number: 25151631.6
(22) Date of filing: 14.01.2025
(51) Int. Cl.: A61B 6/03, A61B 6/08, A61B 6/00

(54) **COMPUTED TOMOGRAPHY SCANNER ARRANGEMENT**

(71) Applicant: Siemens Healthineers AG, 91301 Forchheim (DE)
(72) Inventor: Britzen, Stefan, 91054 Buckenhof (DE); Noack, Olivia, 95444 Bayreuth (DE)
(74) Representative: Siemens Healthineers Patent Attorneys

(57) **Abstract**

Computed tomography scanner arrangement (10, 20, 30), comprising at least one laser arrangement (11, 21, 31) arranged on an inner side of a gantry unit (13, 23, 33) of the computed tomography scanner arrangement (10, 20, 30); wherein the at least one laser arrangement (11, 21, 31) is configured to mark at least one object entry point on an object (13, 23, 33) arranged on a patient table (14, 24, 34) in the gantry unit (12, 22, 32), based on data from at least one control scan of the object (13, 23, 33).

## Description

The present disclosure relates to a computed tomography scanner arrangement, a computer-implemented method controlling at least one first drive mechanism of a laser arrangement causing the laser beam to rotate in at least one axial plane with respect to a gantry unit of a computed tomography scanner arrangement, a corresponding computer program element and a use of data from at least one control scan of an object.

For some medical procedures, like biopsies, drainages, ablations or vertebroplasties and kyphoplasties, it is necessary to bring objects, e.g. a needle-shaped medical equipment into a patient's body. For such procedures, it is common use, that the procedure is supported with repeated online computed tomography imaging for controlling a current position of the object in the patient. Such so called computed tomography-guided interventions represent an established clinical field. Despite the various clinical applications from biopsy over drainages towards more therapeutic procedures involving pain therapy, marker placement and ablations, the workflow is basically comparable for all kinds of computed tomography-guided interventions. In a first step, a patient is positioned on a patient table of a computed tomography scanner arrangement, patient table and the patient are then moved into a patient tunnel of the computed tomography scanner arrangement and a control scan is acquired. Based on this control scan, an object path, e.g. a needle path, from a skin entry point to target lesion is planned. Subsequently, a skin entry point, e.g. needle entry point, is marked on the patient, and the area around this skin entry point is sterilized. Finally, the object is advanced, typically in an iterative so-called step and shoot approach, where several control scans are performed after each movement step towards the target lesion. Once the object has reached its target lesion, the procedure itself can be performed, e.g. steroid injection, sample extraction etc. An important aspect in practice is to mark a skin entry point and also an object orientation, e.g. a needle entry point and a needle orientation, to the patient based on the images/data of a control scan.

In this context, it has become apparent that there is a further need to provide a solution for providing/marking a skin entry point and an object orientation, e.g. a needle entry point and needle orientation, to the patient based on the images of a control scan.

It is therefore an object of the present disclosure to provide a solution for marking a skin entry point and an object orientation, e.g. a needle entry point and needle orientation, to the patient based on the images of a control scan.

These and other objects, which become apparent upon reading the following description, are solved by the subject matters of the independent claims. The dependent claims refer to preferred embodiments of the present disclosure.

In one aspect of the present disclosure, a computed tomography scanner arrangement is disclosed, comprising:
at least one laser arrangement arranged on an inner side of a gantry unit of the computed tomography scanner arrangement; and wherein the at least one laser arrangement is configured to mark at least one object entry point on an object, e.g. a patient, arranged on a patient table in the gantry unit, based on data from at least one control scan of the object.

The term laser arrangement is to be understood broadly in the present context and includes, in particular, arrangements with at least one laser diode configured to direct at least one laser beam onto an object arranged on the patient table of the computed tomography scanner arrangement. The laser beam may be a point-laser beam, in particular a pencil beam, or a fan beam. The term inner side of the gantry unit is also to be broadly understood in the present case. In this respect, the laser arrangement may be arranged on the front of the gantry unit, on its cover or integrated into the gantry unit, for example, in the radiation ring or close by behind a transparent plastic cover. As will be explained in the following, the computed tomography scanner arrangement may also include several laser arrangements, which may have different degrees of freedom with respect to the orientation of the laser beam and with respect to the mobility of the laser arrangement itself. The degrees of freedom of the laser arrangement explained in the present disclosure may be combined at option in preferred embodiments, depending on the respective use case.

In other words, it is proposed that at least one laser arrangement, which is arranged on an inner side of the gantry unit, be provided for visualizing a skin entry point, e.g. a needle entry point. In preferred embodiments of the present disclosure, also an object orientation, e.g. a needle orientation, may be visualized.

In an embodiment of the computed tomography scanner arrangement, the at least one laser arrangement comprises a first drive mechanism for causing the laser to rotate in at least one axial plane with respect to the gantry unit. This allows the laser beam to be moved in the axial plane in order to mark a skin entry point in an area on a patient. The axial plane is a plane that preferably corresponds to the scan plane and is preferably orthogonal to the system axis through the gantry unit.

In an embodiment of the computed tomography scanner arrangement, the computed tomography scanner arrangement comprises at least two laser arrangements, which are arranged at a distance from one another on the inner side of the gantry unit of the computed tomography scanner arrangement. The two laser arrangements are preferably arranged at an angular distance on the inner side of the gantry unit of between 20° and 180°, preferably between 40° and 120° and particularly preferably at an angular distance of approximately 90°, and wherein the two laser arrangements are preferably arranged in an upper area of the gantry unit. By having two laser arrangements set up at different positions, different areas of a patient can be targeted by the laser beams so that a skin entry point can be visualized on the entire patient.

In an embodiment of the computed tomography scanner arrangement, the patient table is configured to be moved in a direction parallel to a system axis through the gantry unit and wherein the patient table is further configured to position the object in the gantry unit such that the object entry point is located in the axial plane of the at least one laser arrangement. The patient table is preferably height-adjustable.

In an embodiment of the computed tomography scanner arrangement, the computed tomography scanner arrangement comprises a guiding arrangement which is arranged on the gantry unit and on which at least one laser arrangement is arranged in a movable manner, wherein the laser arrangement is preferably movable in a circular manner in an angular range of up to 200°, preferably in an angular range of up to 180°. In an example, the guiding arrangement is provided by means of a rail arrangement attached at the inner side of the gantry unit and wherein the at least one laser arrangement is preferably moved on the guiding arrangement by means of at least one pace motor. This makes it possible to cover a wide area on the patient with just one laser arrangement.

In an embodiment of the computed tomography scanner arrangement, the at least one laser arrangement comprises a second drive mechanism for causing the laser to rotate in at least one plane perpendicular to the axial plane with respect to the gantry unit. By means of such a second drive mechanism, the laser beam can be aligned in such a way that not only an entry point but also an object orientation, e.g. a needle orientation, can be indicated by the laser beam. The at least one plane perpendicular to the axial plane may be parallel to the system axis.

In an embodiment of the computed tomography scanner arrangement, the gantry unit is configured to be tilted based on data from at least one control scan of the object. This makes it possible to align the laser beam as well, because the orientation of the laser beam can also be adjusted by tilting the gantry unit. The laser beam can thus be provided on the one hand by a further degree of freedom of the laser arrangement by means of the second drive mechanism and/or by a tilting of the gantry unit. In both cases, it is possible not only to mark an entry point with the laser beam on a patient but also to visualize the orientation of the object with the laser beam.

A further aspect of the present disclosure relates to a computer-implemented method for providing control data for controlling at least one first drive mechanism of a laser arrangement causing the laser to rotate in at least one axial plane with respect to a gantry unit of a computed tomography scanner arrangement as described above, comprising:
providing data from at least one control scan of an object arranged on a patient table in the gantry unit of the computed tomography scanner arrangement;
providing control data for the at least one first drive mechanism of the laser arrangement causing the laser to mark at least one object entry point on the object arranged on the patient table in the gantry unit.

In an embodiment of the computer-implemented method for providing control data, the method is further comprising:
providing control data for at least one second drive mechanism of the laser arrangement for causing the laser to rotate in at least one plane perpendicular to the axial plane with respect to the gantry unit;
providing control data for at least one patient table movement mechanism for moving a patient table in a direction parallel to a system axis through the gantry unit and/or to move the patient table and/or to adjust the height position of the patient table; and/or
providing control data for at least one gantry tilt mechanism for tilting the gantry unit.

A further aspect of the present disclosure relates to a computer program element with instructions, which, when executed on computing devices of a computing environment, is configured to carry out the steps of the above-described computer-implemented method in an above-described computed tomography scanner arrangement. The computer program element might be stored on a computing unit of a computing device, which might also be part of an embodiment. This computing unit may be configured to perform or induce performing of the steps of the method described above. Moreover, it may be configured to operate the components of the above-described system. The computing unit can be configured to operate automatically and/or to execute the orders of a user. The computing unit may include a data processor. A computer program may be loaded into a working memory of a data processor. The data processor may thus be equipped to carry out the method according to one of the preceding embodiments. This exemplary embodiment of the present disclosure covers both, a computer program that right from the beginning uses the present disclosure and computer program that by means of an update turns an existing program into a program that uses the present disclosure. Moreover, the computer program element might be able to provide all necessary steps to fulfill the procedure of an exemplary embodiment of the method as described above. According to a further exemplary embodiment of the present disclosure, a computer readable medium, such as a CD-ROM, USB stick, a downloadable executable or the like, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems. However, the computer program may also be presented over a network like the World Wide Web and can be downloaded into the working memory of a data processor from such a network. According to a further exemplary embodiment of the present disclosure, a medium for making a computer program element available for downloading is provided, which computer program element is arranged to perform a method according to one of the previously described embodiments of the present disclosure.

A further aspect of the present disclosure relates to a use of data from at least one control scan of an object arranged on a patient table in a gantry unit of a computed tomography scanner arrangement for providing control data as described above.

This and embodiments described herein relate to the methods, the systems, the apparatuses, the computer program element, the computer-readable storage medium, the use lined out above and vice versa. Advantageously, the benefits provided by any of the embodiments and examples equally apply to all other embodiments and examples and vice versa.

The term data as used herein is to be understood broadly in the present case and represents any kind of data. Data may be single numbers/numerical values, a plurality of a numbers/numerical values, a plurality of a numbers/numerical values being arranged within a list, 2 dimensional maps or 3 dimensional maps, but are not limited thereto.

The term providing as used herein is to be understood broadly in the present case and represents any providing, receiving, querying, measuring, calculating, determining, transmitting of data, but is not limited thereto. Data may be provided by a user via a user interface, depicted/shown to a user by a display, and/or received from other devices, queried from other devices, measured other devices, calculated by other device, determined by other devices and/or transmitted by other devices.

In the following particularly preferred embodiments are disclosed, which may be combined with the above-disclosed methods, systems, apparatuses, devices and/or use cases. The embodiments described herein may be combined unless specifically noted otherwise. Features and advantages described with respect to one aspect of the disclosure may be applied to other aspects of the disclosure.

In the following, the present disclosure is further described with reference to the enclosed figures:
- Figure 1: illustrates an example of a computed tomography scanner arrangement according to an embodiment of the present disclosure;
- Figure 2: illustrates a further example of a computed tomography scanner arrangement according to an embodiment of the present disclosure;
- Figure 3: illustrates a further example of a computed tomography scanner arrangement according to an embodiment of the present disclosure; and
- Figure 4: illustrates a flow diagram of a computer-implemented method for providing control data according to an embodiment of the present disclosure.

The following embodiments are mere examples for implementing the method, the system, disclosed herein and shall not be considered limiting.

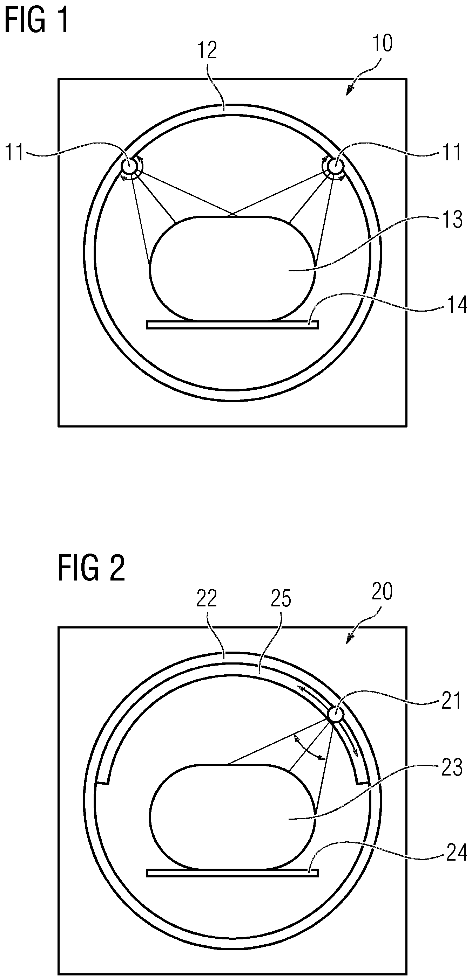

Figure 1 illustrates an example of a computed tomography scanner arrangement 10 according to an embodiment of the present disclosure. In this embodiment, two stationary laser arrangements 11, with two laser diodes, are provided. In the preferred embodiment, the laser arrangements 11 are integrated in a gantry ring 12 of a gantry unit of the computed tomography scanner arrangement 10. As indicated in Figure 1, in the shown cross-sectional view, the laser arrangements 11 are located roughly at 2 and 10 o'clock positions such that a majority of a patient 13 can be marked by the laser arrangements 11, e.g. both sides and the back/front depending on the patient's 13 positioning. Despite being stationary with regards to the gantry ring 12, the laser arrangements 11, e.g. the laser diodes, can be rotated around their own axes in scan plane-only by means of a first drive mechanism (not shown). An example of an area that may be covered by each one of the laser arrangements 11 is indicated in Figure 1. The positioning of both laser arrangements 12 in combination with the rotation allows to cover the entire patient 13 width within a scan plane of the computed tomography scanner arrangement 10. Moreover, a patient table 14 onto which the patient 13 is arranged is configured to be moved in a direction parallel to a system axis through the gantry ring 12 and wherein the patient table 14 is further configured to position the patient 13 in the gantry unit such that a needle entry point is located in the axial plane of one of the laser arrangements 11. As a result, a needle entry point can be marked onto the patient's skin via one of both laser arrangements 11. However, in this embodiment no indication for a needle orientation is provided.

Figure 2 illustrates a further example of a computed tomography scanner arrangement 20 according to an embodiment of the present disclosure. Also here, a patient 23 is arranged on a patient table 24. As indicated in Figure 2, a laser arrangement 21 is movably arranged at a guiding arrangement 25, e.g. a rail arrangement 25, which in turn is arranged on the inner side of the gantry unit/ring 22 and on which the laser arrangement 21 is movable in a circular manner in an angular range of up to 180°. In this embodiment, the laser arrangement 21, e.g. the laser diode, can be rotated around their own axes in scan plane-only by means of a first drive mechanism (not shown). As a result, by means of this embodiment of a computed tomography scanner arrangement 20, a needle entry point can be marked onto the patient's skin via the laser arrangement 21. However, in this embodiment no indication for a needle orientation is provided.

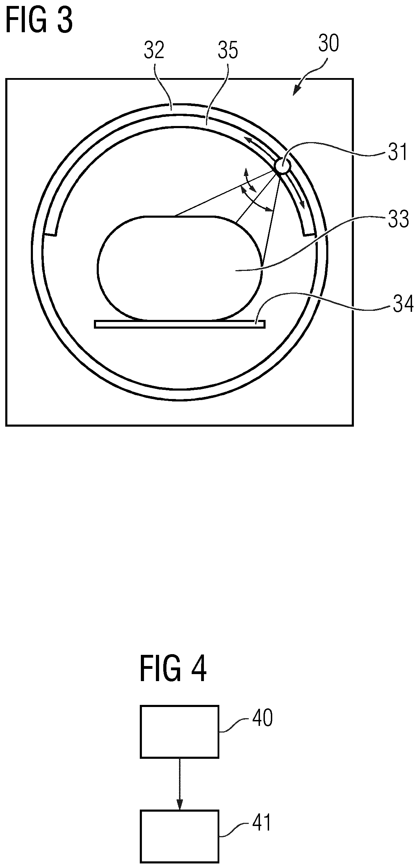

Figure 3 illustrates a further example of a computed tomography scanner arrangement 30 according to an embodiment of the present disclosure. Also here, a patient 33 is arranged on a patient table 34. In addition to the embodiment shown in Figure 2, a laser arrangement 31, e.g. the laser diode, can further be rotated in at least one plane perpendicular to the axial plane with respect to the gantry unit/ring 33 by means of a second drive mechanism (not shown). Moreover, the laser arrangement 31 is also movably arranged at a guiding arrangement 35, e.g. a rail arrangement 35, which in turn is arranged on the inner side of the gantry unit/ring 32 and on which the laser arrangement 31 is movable in a circular manner in an angular range of up to 180°. As a result, by means of this embodiment of a computed tomography scanner arrangement 30, a needle entry point can be marked onto the patient's skin via the laser arrangement 31 and also an object orientation, e.g. a needle orientation, can be indicated by the laser beam, the needle orientation in three dimensions, so all directions, inclusive the Z-angulation.

As an additional option to the preferred embodiments shown in Figures 1 to 3, the gantry unit/ring 12, 22, 32 may be configured to be tilted based on data from at least one control scan of the patient 13, 23, 33. This makes it possible to align the laser beam as well, because the orientation of the laser beam can also be adjusted by tilting the gantry unit/ring 12. The laser beam can thus be provided on the one hand by a further degree of freedom of the laser arrangement 11, 21, 31 by means of the second drive mechanism and/or by a tilting of the gantry unit/ring 12, 22, 33. In both cases, it is possible not only to mark an entry point with the laser beam on a patient 13, 23, 33, but also to visualize the orientation of the object/needle with the laser beam. Thus, the gantry tilt allows to indicate also the z-angulation, the laser rotation in scan-plane allows for in-plane angulation and thus, a three-dimensional indication may be provided.

Figure 4 illustrates a flow diagram of a computer-implemented method for providing control data for controlling at least one first drive mechanism of a laser arrangement 11, 21, 31 causing the laser beam of the laser arrangement 11, 21, 31 to rotate in at least one axial plane with respect to the gantry ring/unit 12, 22, 32 of a computed tomography scanner arrangement 10, 20, 30. In a first step 40, data from at least one control scan of an object/patient 13, 23, 33 arranged on a patient table 14, 24, 34 in the gantry unit 12, 22, 32 of the computed tomography scanner arrangement 10, 20, 30 are provided. In a further step 41, control data for the at least one first drive mechanism of the laser arrangement 11, 21, 31 causing the laser to mark at least one object entry point on the object/patient 13, 23, 33 arranged on the patient table 14, 24, 34 in the gantry unit 12, 22, 32 are provided.

The present disclosure has been described in conjunction with a preferred embodiment as examples as well. However, other variations can be understood and effected by those persons skilled in the art and practicing the claimed invention, from the studies of the drawings, this disclosure and the claims. Notably, in particular, the any steps presented can be performed in any order, i.e. the present invention is not limited to a specific order of these steps. Moreover, it is also not required that the different steps are performed at a certain place or at one node of a distributed system, i.e. each of the steps may be performed at a different node using different equipment/data processing units.

As used herein "determining" also includes "estimating, calculating, initiating or causing to determine", "generating" also includes "initiating or causing to generate" and "providing" also includes "initiating or causing to determine, generate, select, send, query or receive".

In the claims as well as in the description the word "comprising" does not exclude other elements or steps and the indefinite article "a" or "an" does not exclude a plurality. A single element or other unit may fulfill the functions of several entities or items recited in the claims. The mere fact that certain measures are recited in the mutual different dependent claims does not indicate that a combination of these measures cannot be used in an advantageous implementation. Furthermore, it should be noted that, regardless of the grammatical gender of a particular term, it includes persons with male, female or other gender identities.

## Claims

1. Computed tomography scanner arrangement (10, 20, 30), comprising:
at least one laser arrangement (11, 21, 31) arranged on an inner side of a gantry unit (13, 23, 33) of the computed tomography scanner arrangement (10, 20, 30); wherein the at least one laser arrangement (11, 21, 31) is configured to mark at least one object entry point on an object (13, 23, 33) based on data from at least one control scan of the object (13, 23, 33), the object (13, 23, 33) being arranged on a patient table (14, 24, 34) in the gantry unit (12, 22, 32).

2. Computed tomography scanner arrangement (10, 20, 30) according to claim 1, wherein the at least one laser arrangement (11, 21, 31) comprises a first drive mechanism for causing a laser beam of the at least one laser arrangement (11, 21, 31) to rotate in at least one axial plane with respect to the gantry unit (12, 22, 32).

3. Computed tomography scanner arrangement (10, 20, 30) according to claim 1 or claim 2, wherein the computed tomography scanner arrangement (10, 20, 30) comprises at least two laser arrangements (11, 21, 31), which are arranged at a distance from one another on the inner side of the gantry unit of the computed tomography scanner arrangement (10, 20, 30).

4. Computed tomography scanner arrangement (10, 20, 30) according to claim 3, wherein the two laser arrangements (11, 21, 31) are arranged at an angular distance on the inner side of the gantry unit (12, 22, 32) of between 20° and 180°, preferably between 40° and 120° and particularly preferably at an angular distance of approximately 90°, and wherein the two laser arrangements (11, 21, 31) are preferably arranged in an upper area of the gantry unit (12, 22, 32).

5. Computed tomography scanner arrangement (10, 20, 30) according to any one of the preceding claims, wherein the patient table (14, 24, 34) is configured to be moved in a direction parallel to a system axis relative to the gantry unit (12, 22, 32) and wherein the patient table (14, 24, 34) is further configured to position the object (13, 23, 33) in the gantry unit (12, 22, 32) such that the object entry point is located in the axial plane of the at least one laser arrangement (11, 21, 31).

6. Computed tomography scanner arrangement (10, 20, 30) according to any one of the preceding claims, wherein the patient table (14, 24, 34) is height-adjustable.

7. Computed tomography scanner arrangement (10, 20, 30) according to any one of the preceding claims, wherein the computed tomography scanner arrangement (10, 20, 30) comprises a guiding arrangement (25, 35) which is arranged on the gantry unit (12, 22, 32) and on which the at least one laser arrangement (11, 21, 31) is arranged in a movable manner, wherein the at least one laser arrangement (11, 21, 31) is preferably movable in a circular manner in an angular range of up to 200°, preferably in an angular range of up to 180°.

8. Computed tomography scanner arrangement (10, 20, 30) according to claim 7, wherein the guiding arrangement (25, 35) is provided by means of a rail arrangement (25, 35) attached at the inner side of the gantry unit (12, 22, 32) and wherein the at least one laser arrangement (11, 21, 31) is preferably moved on the guiding arrangement (25, 35) by means of at least one pace motor.

9. Computed tomography scanner arrangement (10, 20, 30) according to any one of the preceding claims, wherein the at least one laser arrangement (11, 21, 31) comprises a second drive mechanism for causing the laser beam of the at least one laser arrangement (11, 21, 31) to rotate in at least one plane perpendicular to the axial plane with respect to the gantry unit (12, 22, 32).

10. Computed tomography scanner arrangement (10, 20, 30) according to any one of the preceding claims, wherein the gantry unit (12, 22, 32) is configured to be tilted based on data from at least one control scan of the object (13, 23, 33).

11. Computer-implemented method for providing control data for controlling at least one first drive mechanism of a laser arrangement (11, 21, 31) causing a laser beam of the at least one laser arrangement (11, 21, 31) to rotate in at least one axial plane with respect to the gantry unit (12, 22, 32) of a computed tomography scanner arrangement (10, 20, 30) according to one of the preceding claims, comprising:
providing (40) data from at least one control scan of an object (13, 23, 33) arranged on a patient table (14, 24, 34) in the gantry unit (12, 22, 32) of the computed tomography scanner arrangement (10, 20, 30);
providing (41) control data for the at least one first drive mechanism of the laser arrangement (11, 21, 31) causing the laser beam of the at least one laser arrangement (11, 21, 31) to mark at least one object entry point on the object (13, 23, 33) arranged on the patient table (14, 24, 34) in the gantry unit (12, 22, 32).

12. Computer-implemented method for providing control data further comprising:
providing control data for at least one second drive mechanism of the laser arrangement (11, 21, 31) for causing the laser beam of the at least one laser arrangement (11, 21, 31) to rotate in at least one plane perpendicular to the axial plane with respect to the gantry unit (12, 22, 32);
providing control data for at least one patient table movement mechanism for moving a patient table (14, 24, 34) in a direction parallel to a system axis relative to the gantry unit (12, 22, 32) and/or to move the patient table (14, 24, 34) and/or to adjust the height position of the patient table (14, 24, 34); and/or
providing control data for at least one gantry tilt mechanism for tilting the gantry unit (12, 22, 32).

13. Computer program element with instructions, which, when executed on computing devices of a computing environment, is configured to carry out the steps of the computer-implemented method according to claim 11 or claim 12 in a computed tomography scanner arrangement (10, 20, 30) according to any one of the claims 1 to 10.

14. Use of data from at least one control scan of an object (13, 23, 33) arranged on a patient table (14, 24, 34) in a gantry unit of a computed tomography scanner arrangement (10, 20, 30) for providing control data according to claim 11 or claim 12.
